# FASCICULE DE BREVET EUROPEEN

(11) **EP 0 782 563 B1**
(45) Date de publication et mention de la délivrance du brevet: **23.06.1999**
(21) Numéro de dépôt: 95932034.2
(22) Date de dépôt: 20.09.1995
(51) Int. Cl.: C07C 237/46, A61K 49/04

(54) **COMPOSES POLYIODES, LEUR PREPARATION ET LEUR APPLICATION EN RADIOLOGIE X**
IODO-VERBINDUNGEN, IHRE HERSTELLUNG UND VERWENDUNG ALS KONTRASTMITTEL FÜR RÖNTGEN-RADIOLOGIE
POLYIODINATED COMPOUNDS, PREPARATION AND USE THEREOF IN X-RAY RADIOLOGY

(30) Priorité: 22.09.1994 FR 9411329
(43) Date de publication de la demande: 09.07.1997
(73) Titulaire: GUERBET, 93420 Villepinte (FR)
(72) Inventeur: LE LEM, Gael, F-92210 Saint-Cloud (FR); MEYER, Dominique, F-94100 Saint-Maur (FR)
(74) Mandataire: Le Guen, Gérard
(86) Numéro de dépôt international: FR9501212
(87) Numéro de publication internationale: WO9609280

(56) Documents cités:
- EP-A- 0 354 836
- WO-A-92/18167
- WO-A-93/10824
- WO-A-94/21600
- WO-A-95/01966
- US-A- 4 065 553
- US-A- 4 065 554

## Description

La présente invention concerne des composés utilisables comme produits de contraste pour la radiologie par rayons X.

Ces composés, qui comprennent au moins 6 noyaux phényles porteurs de 3 ou 4 atomes d'iode, donnent des solutions aqueuses dont la concentration en atomes d'iode est de l'ordre de celles généralement utilisées en clinique, de 5 à 40 g d'iode pour 100 ml, et qui ont une viscosité acceptable pour une administration parentérale, malgré une masse moléculaire de plus de 3000. Ils sont, en outre, éliminés de la circulation sanguine, après administration intravasculaire, nettement plus lentement que les composés connus, à l'exclusion des polymères sur lesquels sont greffées des molécules iodées comme décrit dans EP A-354 836 et WO 93/10824, mais dont l'utilité en clinique n'est toujours pas confirmée, notamment du fait de la polydispersité de leurs masses moléculaires.

Ainsi, après injection intraveineuse chez l'animal d'une même dose, exprimée en poids d'iode, d'un monomère de l'invention ou d'un produit de contraste couramment utilisé en clinique -comme l'iobitridol ou l'iohexol- on constate dans les premières minutes suivant l'administration que la concentration sanguine en iode décroît beaucoup moins vite pour les composés de l'invention. Par exemple, chez le rat, au bout de 5 minutes, la concentration pour un composé selon l'invention est en général au moins 3 fois plus élevée. Comme, par ailleurs, la présence de groupes hydrophiles correctement répartis sur la molécule assure une bonne solubilité aqueuse à ces composés, supérieure à 25 g d'iode par litre, ils sont utiles comme agents de contraste pour l'imagerie par rayons X du compartiment vasculaire.

Les composés de l'invention sont représentés par la formule I: 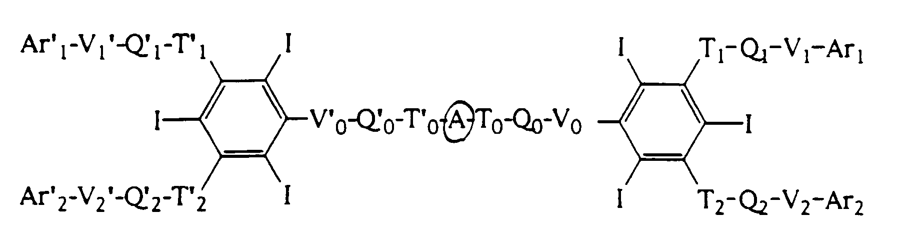 dans laquelle:
- Tᵢ, Vᵢ, T'ᵢ, V'ᵢ, identiques ou différents, avec i = 0, 1, 2, représentent O, CO-ND ou ND-CO D pouvant être H, alkyle en C₁ à C₆, hydroxyalkyle et polyhydroxyalkyle en C₁ à C₆, de préférence en C₁ à C₄;
- Qᵢ et Q'ᵢ identiques ou différents, avec i = 0, 1, 2 représentent alkylène en C₁ à C₆, hydroxyalkylène ou polyhydroxyalkylène en C₁-C₆, et de préférence en C₁ ou C₂;
- Arᵢ et Ar'ᵢ, identiques ou différents, avec 1 = 1, 2, représentent:
   - soit la formule II 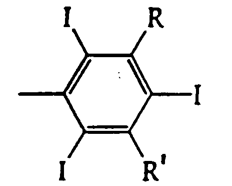 dans laquelle
      R est COOH et R' est CONR'₁R'₂ ou N(R'₁)COR'₂, R'₁ et R'₂ étant H, alkyle en C₁ à C₄, hydroxyalkyle ou polyhydroxyalkyle en C₁ à C₈ et comportant à eux deux plus de 4 hydroxyles et de préférence plus de 6 hydroxyles, ou
      R et R', identiques ou différents, représentent CONR'₁R'₂ ou N(R'₁)COR'₂, R'₁ et R'₂ étant H, alkyle en C₁ à C₄, hydroxyalkyle ou polyhydroxyalkyle en C₁ à C₈ et comportant à eux deux plus de 8 hydroxyles, et mieux au moins 10 hydroxyles,
   - soit la formule III 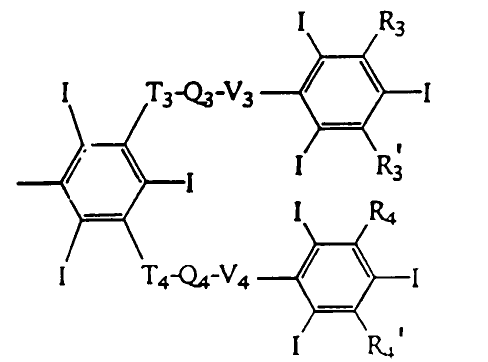 dans laquelle
      les Tᵢ, Qᵢ, Vᵢ avec i = 3, 4, peuvent avoir l'une des significations du cas i = 0 et Rᵢ et R'ᵢ avec i = 3, 4, peuvent avoir l'une des significations de R et R' dans la formule Il ou représentent T-Q-V-Ar avec Ar représenté par la formule Il et T, Q, V pouvant avoir les significations de Tᵢ, Qᵢ, Vᵢ,
      et A est le reste biocompatible d'une molécule aliphatique ou aromatique de masse moléculaire inférieure à 1200, comportant éventuellement des hétéroatomes choisis parmi l'oxygène, l'azote et l'iode, ayant deux liaisons libres susceptibles de donner avec T₀ et T'₀ un groupe amide ou étheroxyde, ainsi que les sels des bases pharmaceutiquement acceptables de ces acides, tels que les sels alcalins, ceux d'amines aliphatiques, comme la N-méthylglucamine ou d'aminoacides tels que la lysine.
      Les groupes alkyles peuvent être linéaires ou ramifiés.

WO 94/21600 appartient à l'état de la technique au sens de l'article 54(3) CBE.

Ce document décrit très généralement des composés polyiodés d'un poids moléculaire unique possédant une concentration moléculaire en iode supérieure à environ 20 % en poids, notamment supérieure à environ 30 % en poids, comportant au moins 9 atomes d'iode et possédant un poids moléculaire supérieur à 2000 et inférieur à environ 50.000, notamment supérieur à 2000 et inférieur à environ 20.000, caractérisés en ce qu'ils possèdent soit une charge électrique globale nulle, soit au moins deux charges anioniques et en ce qu'ils persistent dans le compartiment vasculaire à une valeur égale à au moins environ 30 % du poids de la dose injectée chez un sujet cinq minutes après l'administration intra-vasculaire chez ledit sujet.

Toutefois, cette demande de l'art antérieur ne décrit pas le sous-groupe particulier des composés de l'invention pour lesquels le reste A présente une masse moléculaire inférieure à 1200.
La nature de A n'est pas critique au moment que, selon le cas, A peut former avec T₀ et T'₀ deux groupes amide et/ou étheroxyde.

Parmi les restes A aliphatiques convenant, on peut citer les dérivés
- de diamines, telles que H₂N-(CH₂)n-NH₂ avec n = 2 à 4, la chaîne alkylène étant éventuellement interrompue par un atome d'oxygène ou un groupe amide et pouvant porter un ou plusieurs hydroxyles;
- ou de diacides, tels que les acides fumarique, succinique, ou ceux de formule HOOC-(CH₂)n-COOH dans laquelle n = 1 à 4
- ou de dialcools, tels que l'éthylèneglycol,
- ou d'un aminoacide tel que l'acide γ-aminobutyrique.

Parmi les restes A aromatiques convenant, on peut citer le noyau phényle, substitué ou non, éventuellement iodé, de telle sorte que le reste T'₀-A-T₀ pourra être obtenu à partir des acides phényldicarboxyliques, des phénylènediamines ou des acides aminophénylcarboxyliques, éventuellement iodés, notamment l'acide tétra-iodophtalique et ses isomères, l'acide 3-acétylaminotriiodoisophtalique, la p-phénylènediamine diiodée et les acides hydroxybenzoïques iodés.

Des restes comportant 2 noyaux phényles sont d'autres exemples de A, tels que ceux dérivés de l'acide 4,4'-biphényldicarboxyique ou du bisphénol.

On préfère les composés dans lesquels les deux groupes substituant A sont identiques et plus particulièrement ceux dans lesquels les substituants Tᵢ-Qᵢ-Vᵢ-Arᵢ sont identiques.

Il est aussi souhaitable, étant donné l'application des composés comme produits de contraste, qu'ils comportent le maximum d'atomes d'iode et on préfère les composés dont A contient des atomes d'iode sur un noyau phényle.

Les composés pour lesquels Arᵢ et Ar'ᵢ représentent la formule III, Rᵢ et R'ᵢ ayant la signification de R et R' présentent déjà une excellente rémanence vasculaire et ils sont préférés.

Le procédé de préparation des composés de formule I est un autre objet de l'invention,

Ces composés peuvent être préparés en faisant réagir, dès la première étape, le reste A portant les groupes réactifs convenables, avec une molécule qui constitue une partie des groupes le substituant dans la formule I et, par exemple, avec T^{α} ₀-Q₀-V^{α} ₀, ou avec 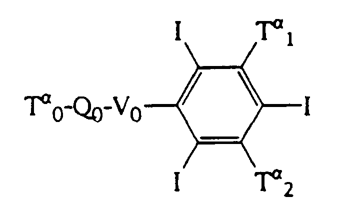 formules dans lesquelles les exposants α signifient que le groupe est un groupe fonctionnel précurseur du groupe figurant dans la formule I et, par exemple, lorsque T₁ est CONH, T₁^{α} est COOH ou COCI et lorsque T₁ est O, T₁^{α} est OH, halogène ou sulfonate.

On fait ensuite réagir sur le composé obtenu, une autre partie des groupes substituants pour obtenir, de proche en proche, par des réactions d'amidification ou d'éthérification classiques, le composé de formule I.

Néanmoins, on préfère préparer d'abord les groupes substituants de formule 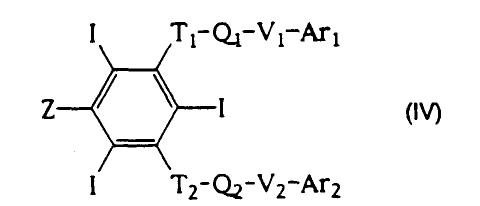 dans laquelle les Tᵢ, Qᵢ, Vᵢ, Arᵢ ont leurs significations de la formule I et Z représente soit Tα₀-Q₀-V₀, soit V^{α}₀, auquel cas, lors de la dernière étape, on fera réagir un reste A sur lequel on aura préalablement greffé T₀-Q₀-V^{β}₀, V^{β}₀ étant un groupe fonctionnel qui, par réaction avec V^{α}₀, donne V₀.

La préparation du composé de formule IV comprend:
1) la réaction du composé de formule 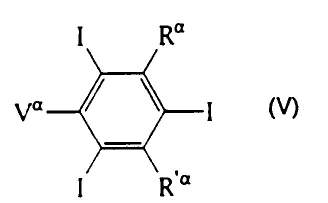 dans laquelle R^{α}, R'^{α}, et V^{α} représentent COCl ou NH₂ ou encore V^{α} représente OH, selon la nature de Rᵢ, R'ᵢ et Vᵢ dans la formule IV,
   avec, lorsque les R^{α} représentent COCl, un aminoalcool de formule HNR'₁R'₂, et lorsque les R^{α} représentent NH₂ avec R'₂COCl ou R_{'2}COOH, cette réaction pouvant être suivie de l'alkylation de l'amide obtenu, par exemple avec R'₁ CI;
2) la réaction du produit obtenu avec T^{α}-Q-V^{β}, T^{α} et -V^{β} étant des groupes précurseurs de Tᵢ et Vᵢ,
   l'ordre des réactions 1 et 2 pouvant être inversé;
3) la réaction du produit obtenu sur 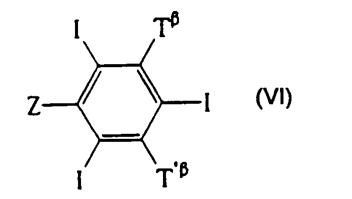 l'exposant β désignant des groupes précurseurs de T, à savoir T₁ et T₂ lorsque dans le composé de formule I, Arᵢ représente la formule II, ou de T₃ et T₄ lorsque Arᵢ représente la formule III,
   et Z représente soit un groupe précurseur T^{β}ᵢ-Qᵢ-Vᵢ, soit un groupe précurseur V_{iβ}, auquel cas le composé obtenu devra réagir avec T^{β}ᵢ-Qᵢ- V^{β}ᵢ avant l'étape suivante;
4) éventuellement, dans le cas où Arᵢ de la formule I représente la formule III, de nouveau la réaction de T-Q-V-Ar ainsi obtenu sur un composé de formule VI dans lequel T^{β} et T'^{β} désignent les précurseurs de T₁ et T₂;
5) la réaction du groupe de formule IV sur A portant un groupe précurseur de T₀ suivie éventuellement de la salification des groupes acides carboxyliques.

Etant donné la nature des groupes R, T, V, les réactions mises en oeuvre au cours de ce procédé sont des réactions d'amidification ou d'éthérification classiques, sachant que la réactivité des groupes fonctionnels situés sur un noyau phényle, lorsque les deux atomes de carbone adjacents portent un atome d'iode, est faible.

Ainsi, les amides sont préparés par action du chlorure d'acide sur l'amine convenable, dans un solvant aprotique dans lequel ils sont insolubles, de préférence en présence d'un composé susceptible de fixer l'acide chlorhydrique libéré, tel qu'une amine tertiaire, par exemple, la triéthylamine ou la tributylamine; ils peuvent aussi être obtenus par action de l'acide sur l'amine en solution, en présence d'un agent de couplage, tels ceux utilisés dans la chimie des peptides, comme le N,N'-dicyclohexylcarbodiimide, le 1-(3-diméthylaminopropyl)-3-éthylcarbodiimide ou la 1-éthoxycarbonyl-2-éthoxy-1,2-dihydroquinoléine éventuellement, en présence d'un activateur, l'hydroxybenzotriazole ou le N-hydroxysuccinimide.

Les fonctions amines ne devant pas réagir sont bloquées, par exemple sous forme de phtalimide.

Les groupes étheroxydes sont préparés par action d'un halogénure ou d'un sulfonate sur l'alcool ou le phénol convenable en présence d'une base forte, de façon connue en soi.

Au cours de ces réactions, les fonctions alcools peuvent être avantageusement protégées sous forme d'ester.

Parmi les noyaux phényles triiodés de départ, on peut citer l'acide 3,5-diamino 2,4,6-triiodobenzoïque décrit dans DE(East) 43,994 ou GB 791,997 ou 782,313 et l'acide 4-hydroxy 2,4,6-triiodo téréphtalique décrit dans Chem. Abs. 69 86643-6.

Leurs chlorures d'acide, préparés par action de SOCl₂, sont mis à réagir soit avec l'aminoalcool convenable, dont les hydroxyles ont été éventuellement protégés par estérification, soit avec l'amine pour créer le pont entre ce noyau et A ou un autre noyau phényle; dans une seconde étape, les fonctions phénol ou aniline sont mises en réaction dans les conditions classiques.

Les aminoalcools HNR'₁R'₂ dans lesquels R'₁ et R'₂ comportent chacun au moins un hydroxyle et à eux deux au moins 6 ou mieux plus de 8 hydroxyles sont préférés.

Parmi ceux-ci, on peut citer ceux avec
- R'₁ = CH₂(CHOH)₄-CH₂OH et R'₂ = CH₂-(CHOH)₄-CH₂OH ou R'₂ = CH₂-CH₂OH ou R'₂ = CH₂-CHOH-CH₂OH qui sont commercialisés, 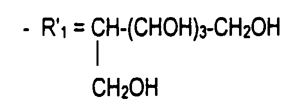 et R'₂ = CH₂-CHOH-CH₂OH décrit dans EP-A-558395,
- R'₁ = R'₂ = CH₂-(CHOH)₃-CH₂OH décrit dans J. Org. Chem. 35(2) 464-7 (1970),
- R'₁ = R'₂ = CH₂-(CHOH)₂-CH₂OH décrit dans US 4,661,646.

D'autres aminoalcools peuvent être préparés par disubstitution de la benzylamine avec un dérivé halogéné ou sulfoné de l'alcool convenable suivie de la débenzylation du composé obtenu, notamment par action de H₂; on peut aussi faire réagir un aldéhyde hydroxylé comme un saccharide sur un aminoalcool primaire et réduire l'imine obtenue par action de H₂.

On obtient ainsi les composés avec 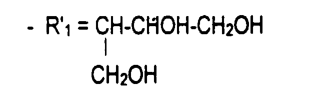 ou - R'₁ = CH₂-(CHOH)₃-CH₂OH
R'₂ = CH₂-CHOH-CH₂OH
ou R'₂ = CH₂-(CHOH)₂-CH₂OH
R'₂ = CH₂-CHOH-CH₂OH ou
R'₂ = CH₂-(CHOH)₂-CH₂OH

Les compositions de produit de contraste radioopaques qui comprennent comme produit absorbant les rayons X au moins l'un des composés de formule I, sont un autre objet de l'invention. Ces compositions peuvent être administrées par voie orale, rectale ou parentérale, notamment par voie intraveineuse, intra-artérielle, intrabronchique ou intra-arachnoïdienne, sous une forme pharmaceutique convenable.

Etant donné leur pharmacocinétique, elles sont particulièrement adaptées à l'imagerie cardiaque et vasculaire.

Les excipients peuvent être ceux habituellement utilisés dans ce domaine du diagnostic radiologique, auxquels peuvent être associés les additifs connus pour ajuster l'osmolalité ou le pH ou diminuer certains effets secondaires connus pour des produits fortement iodés.

Pour l'administration intraveineuse, notamment pour l'observation du compartiment vasculaire, on préfère des solutions aqueuses isoosmolaires, de pH voisin de 7, qui contiennent de 5 g à 40 g d'iode fixé aux noyaux phényles pour 100 ml, dont on administrera de 10 ml à 250 ml.

Dans ce qui suit, on décrit des exemples de composés objet de l'invention et préalablement la préparation de groupes qui seront fixés sur le coeur A.
A) Préparation du composé C₁ de formule VIII: 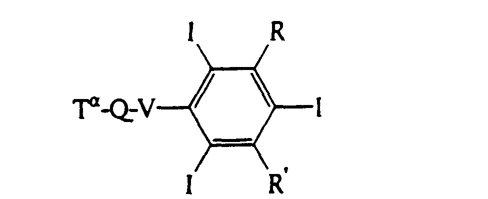 avec R = R' = CON-(CH₂(CHOH)₄CH₂OH)₂
   T^{α}-Q-V = H₂N-CH₂-CO-NH 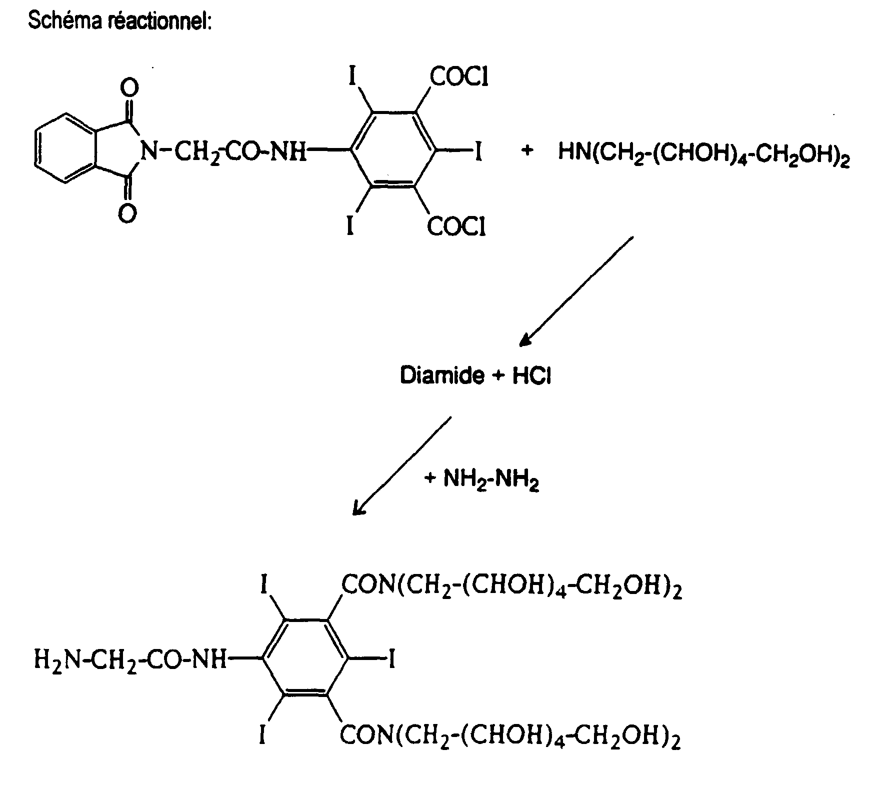
   Etape (a)
      Sous agitation, on introduit peu à peu 258 g de chlorure de N-phtaloylglycine dans une solution à 0° C de 238 g de dichlorure de l'acide 5-amino-2,4,6-triiodoisophtalique dans 1 litre de diméthylacétamide; le mélange est agité plusieurs heures à 0° C puis, après une nuit à température ambiante, il est introduit lentement dans 20 litres d'eau; le précipité formé est isolé et dissous dans 6 litres d'acétate d'éthyle. Cette solution est lavée avec une solution aqueuse de bicarbonate de sodium, puis avec de l'eau, séchée, puis concentrée. On obtient 250 g du dichlorure de l'acide 2,4,6-triiodo-5-phtalimidoacétamidoisophtalique.
   Etape (b)
      208 g du dichlorure d'acide ci-dessus, 416 g de l'amine commerciale et 100 ml de triéthylamine, sont dissous dans 2 litres de N-méthylpyrrolidone ou de diméthylacétamide et la solution est maintenue à 70° C pendant 24 heures. Le précipité formé est séparé et le solvant est éliminé par distillation sous pression réduite. Le résidu, dissous dans le minimum d'eau à pH 3, est passé sur une résine échangeuse d'ions cationique (1,5 l d'Amberlite® IRN77 sous forme acide, commercialisée par Rohm et Haas) pour éliminer les impuretés. A une solution du diamide ainsi obtenu dans 1,4 l d'eau, on ajoute 33,2 ml d'hydrate d'hydrazine et on maintient le mélange à 80° C pendant 3 heures. A la température ambiante, on acidifie par addition de 53 ml d'une solution aqueuse d'acide chlorhydrique 10N et on sépare le précipité formé.
      La solution résiduelle est passée sur une colonne de résine échangeuse d'ions anionique comprenant 1 litre d'Amberlite® IRA67 basique puis sur une colonne de 150 ml d'Amberlite® IRC50 sous forme acide puis greffée sur 4 litres d'Amberlite® 200C d'où le produit est élué par une solution aqueuse de NH₄OH. L'éluant est concentré sous pression réduite pour donner, avec un rendement de 70%, le produit cherché.
      Chromatographie HPLC: colonne LiCrosphère® C 18; 5 µm (Merck) ; I 25 cm; d = 4 mm.
      Eluant(*): CH₃CN/P.I.C.® B8 0,05 M (Waters) 5/95; débit: 1 ml/minute.
      Temps de rétention des isomères: 8 minutes environ.
      (*) P.I.C. B8: mélange acide octane sulfonique/méthanol/acétate de calcium/eau.
B) Préparation du composé C₂ de formule IV dans laquelle 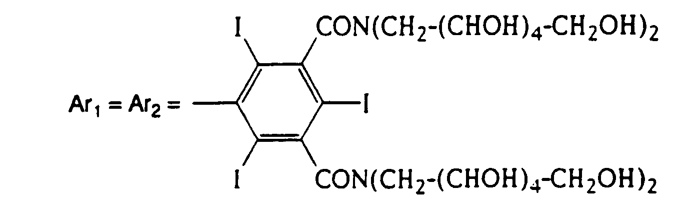 T₁-Q₁-V₁ = T₂-Q₂-V₂ = CONH-CH₂-CONH
   Z = H₂N-CH₂-CONH
   Une solution de 59 g du dichlorure d'acide préparée dans l'étape (a) A, 200 g de l'amine primaire C₁ obtenue en A, et 29,5 ml de tributylamine dans 400 ml de N-méthylpyrrolidone ou de diméthylacétamide est maintenue à 70° C pendant 24 heures. Le solvant est éliminé sous pression réduite et le résidu est chromatographié sur 3 kg de silice silanisée RP2, commercialisé par MERCK (DE) en éluant avec de l'eau ou sur 4 kg d'adsorbant XAD 1600 (Rohm et Haas) en éluant avec un mélange CH₃OH/H₂O.
   Le produit obtenu avec 50% de rendement, est traité par l'hydrate d'hydrazine, comme décrit précédemment, pour donner le composé cherché avec un rendement de 45%.
   Le volume d'élution de ce composé lors d'une filtration sur un gel Superdex® 30 dans une colonne de 16 mm x 60 cm, commercialisée par PHARMACIA dans un tampon de pH = 7,2 comprenant NaCI 0,1 M, NaH₂PO₄ 0,05 M et NaN₃ 0,01 M, avec un débit de 1 ml/minute, et de 102 ml pour un échantillon injecté de 1 mg dans 250 µl de tampon.
   Chromatographie HPLC: colonne Symmetry® C 18; 5 µm (Merck) ; I = 25 cm; d = 4,6 mm.
   Eluant: CH₃CN/KH₂PO₄ 0,01 M (15/85) (sans CH3CN pendant les 5 premières minutes)
   débit: 1 ml/minute.
   Temps de rétention des isomères: 18 minutes environ.
C) Préparation du composé C₃ de formule IV avec
   T₁-Q₁-V₁ = T₂-Q₂-V₂ = CONH-CH₂-CONH Z = H₂N-CH₂-CONH 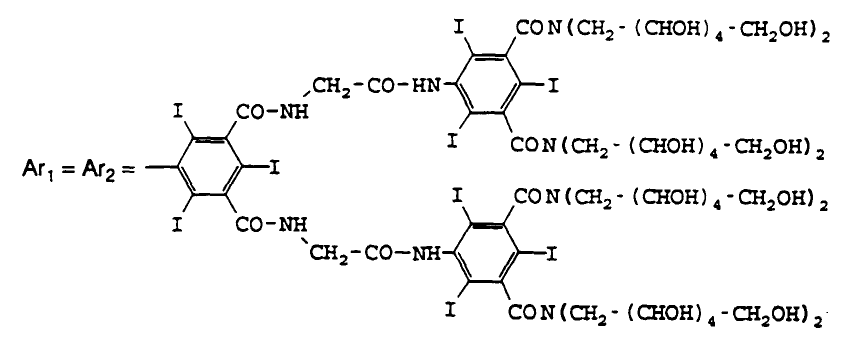
   5,65 g du dichlorure d'acide de l'étape (a) A, 48 g de l'amine primaire C₂ obtenue selon B et 3 ml de tributylamine dans 100 ml de N-méthylpyrrolidone ou de diméthylacétamide sont maintenus à 70° C pendant 24 heures. Après purification comme selon B, le dérivé comportant les 7 noyaux phényles iodés est traité par l'hydrate d'hydrazine pour donner l'amine primaire brute. Celle-ci est purifiée par passage sur des résines échangeuses d'ions sous forme acide et basique comme dans la préparation B. Eventuellement, on peut ensuite effectuer une diaultrafiltration avec une cassette Minisette® de type nova commercialisée par FILTRON TECHNOLOGY Corp. (USA) avec une membrane en polyethersulfone de seuil de coupure 10 kdaltons qui laisse passer le produit cherché. Rendement: 65%.
   Le volume d'élution sur gel de ce produit dans les mêmes conditions opératoires que dans la préparation B est de 91 ml, alors qu'il est de 111 ml sur une colonne Superdex® 75.
   Chromatographie HPLC: colonne Symmetry® C 18; 5 µm (Waters) ; I = 25 cm; d = 4,6mm.
   Eluant: CH₃CN/KH₂PO₄ 0,01 M (15/85) (sans CH₃CN pendant 5 minutes) débit: 1 ml/minute.
   Temps de rétention des isomères: 23 minutes environ.
   Les composés C₂ et C₃ sont pratiquement insolubles dans l'acétone, l'acétonitrile, le tétrahydrofuranne ou les éthers mono et diméthyliques de l'éthylène glycol; il sont solubles dans les amides tels que diméthylacétamide, diméthylformamide ou N-méthylpyrrolidone.
D) Préparation du composé C₄ de formule 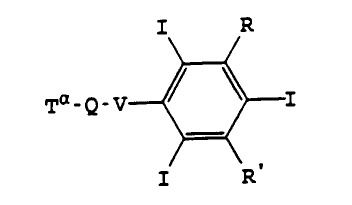 avec R = COOH 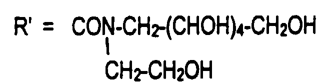 T^{α}-Q-V = H₂N-CH₂-CONH
   (1) 239 g du dichlorure d'acide décrit dans la préparation (a) A, 83 g de 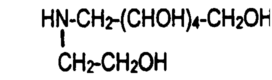 commercialisé par ALDRICH,
      et 39 ml de triéthylamine sont dissous dans 1 litre de N,N-diméthylacétamide. Après 24 heures à température ambiante, on introduit 300 ml d'eau dans le milieu qui est ensuite maintenu pendant 48 heures à 45° C pour hydrolyser le chlorure d'acide résiduel. Les solvants sont alors éliminés par distillation sous pression réduite et le résidu est purifié comme précédemment par passage sur une colonne de 500 ml d'Amberlite® IRN77 et une de 3 kg de silice silanisée RP2.
      On obtient ainsi le monoamide avec un rendement de 40%.
   (2) Ce composé est traité par l'hydrate d'hydrazine et purifié par passage sur des résines échangeuses d'ions acide et basique comme dans la préparation A. Rendement: 70%.
E) Préparation du composé C5 de formule IV avec
   T₁-Q₁-V₁ = T₂-Q₂-V₂ = CONH-CH₂-CONH
   Z = H₂N-CH₂-CONH

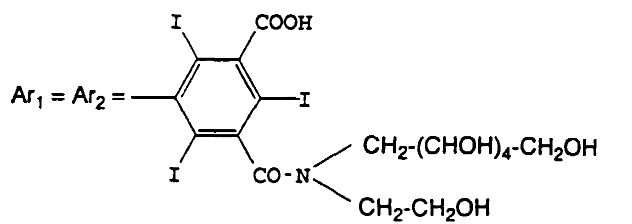

Avec des conditions opératoires analogues à celles utilisées dans la préparation B, on obtient le composé recherché avec 30% de rendement.

Son volume d'élution lors d'une filtration sur gel de Superdex® 30 dans les conditions précédentes est de 92 ml.

Les composés exemplifiés sont caractérisés par leurs temps de rétention dans une chromatographie d'exclusion stérique (CES) sur 4 colonnes, montées en série, commercialisées par SHODEX (JP) sous les références OH paK SB-800 HQ de diamètre 8 mm et de longueur 30 cm, contenant un gel de polyhydroxyméthacrylate: SB-804 (limite d'exclusion = 10⁶ kdaltons; standard: pullulan)+ SB-803 (10⁵)+ SB-802-5 (10⁴)+ SB-802-5; l'éluant est un mélange de solution aqueuse de NaCI 0,16 M et d'acétonibile (70/30 - V/V) - débit 0,8 ml/minute - T = 30° C.

### Exemple 1

Composé No. 1 de formule I, dans laquelle
T₀-Q₀-V₀ = T'₀Q'₀-V'₀ = CONH-CH₂-CONH et 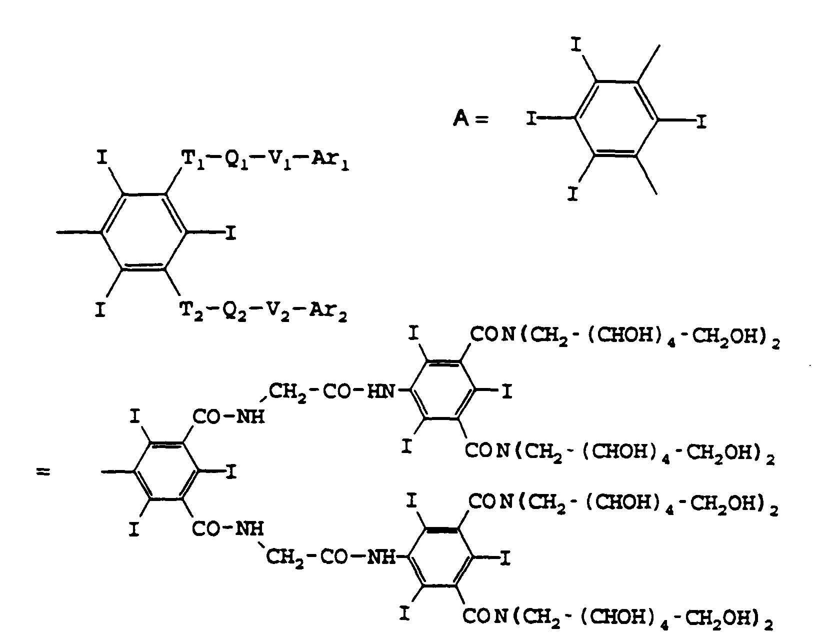
(1) Acide tétraiodoisophtalique:
   On introduit goutte à goutte à -5° C, 30 ml d'une solution aqueuse de 2 g de NaNO₂ dans 60 ml d'une solution de Il g d'acide 5-amino-2,4,6-triiodophényl 1,3-dicarboxylique dans NaOH aqueux 1N. Le milieu est ensuite amené à pH 2 par addition d'une solution aqueuse de H₂SO₄ et maintenu encore 3 heures sous agitation à 5° C avant d'être porté à pH 5 par addition de NaOH aqueux 1N.
   9,6 g de Kl en solution dans 20 ml d'eau sont alors ajoutés goutte à goutte puis le milieu est porté lentement à 45° C et maintenu pendant 2 heures à cette température avant d'être versé sur un mélange glace/HCl.
   Le précipité formé est lavé avec une solution aqueuse de bisulfite de Na puis avec 150 ml de CH₂Cl₂. 11 g de cristaux beiges sont isolés.
   CCM: plaque silice MERCK. Rf = 0,7 (Eluant HCO₂H/CH₃COC₂H₅/C₆H₅CH₃ 2512516).
(2) Dichlorure de l'acide tétraiodoisophtalique:
   Un mélange de 10,4 g du diacide précédent et 150 ml de SOCl₂, avec 0,2 ml de diméthylformamide est maintenu 8 heures à sa température de reflux, puis le milieu est amené à sec par évaporation sous pression réduite. Le résidu est cristallisé dans l'éther de pétrole. On isole 11 g de cristaux beiges.
   CCM: Rf = 0,1 (conditions précédentes).
(3) Composé No. 1
   16 g du composé C₂ obtenu dans la préparation B, 4 g du dichlorure de l'acide tétraiodoisophtalique et 1,5 ml de triéthylamine sont introduits dans 120 ml de diméthylformamide et le milieu réactionnel est maintenu 12 heures à 80° C. le précipité est éliminé par filtration après refroidissement et le solvant est évaporé sous pression réduite. Le résidu, dissous dans 1 litre d'eau, est soumis à une dia-ultrafiltration avec une cassette Minisette®, avec une membrane de seuil de coupure 5 kdaltons pour donner le produit cherché.

### Exemple 2

Composé de formule I, dans laquelle 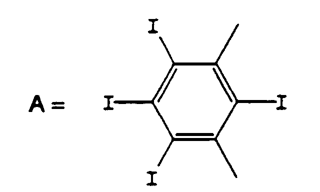 T₀-Q₀-V₀ = T'₀-Q'₀-V'₀ = CONH-CH₂-CONH et
T₁-Q₁-V₁-Ar₁ = T₂-Q₂-V₂-Ar₂ = CONH-CH₂-CONH-Ar avec

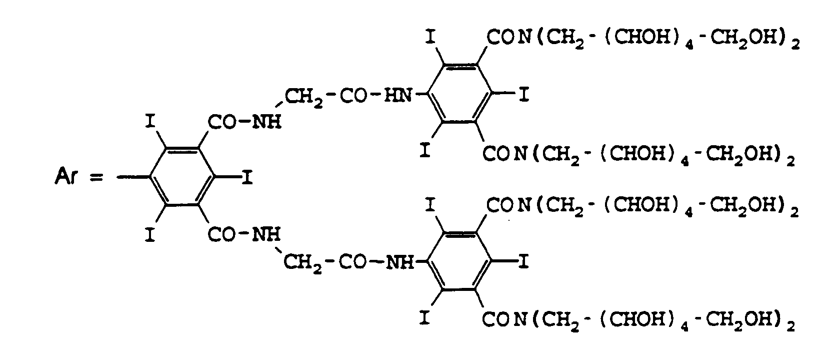

Un équivalent du dichlorure de l'acide tétraiodoisophtalique préparé comme dans l'exemple 1(2) et 2,4 équivalents de l'amine C₃ obtenue dans la préparation C, avec 1 équivalent de tributylamine, sont mis en solution dans la quantité minimale de diméthylacétamide et le mélange est maintenu 10 heures à 80° C avant d'être traité comme à l'exemple 1.

Le produit cherché est isolé après ultrafiltration de sa solution aqueuse dans le même type de cassette qu'à l'exemple 1 mais comportant une membrane dont le seuil de coupure est de 10 kdaltons.

### Exemple 3

Composé No. 3 de formule I, dans laquelle
T₀-Q₀-V₀ = T'₀-Q'₀-V'₀ = CONH-CH₂-CONH et 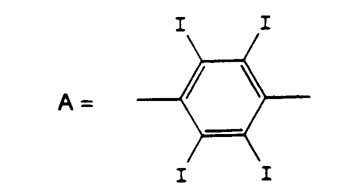 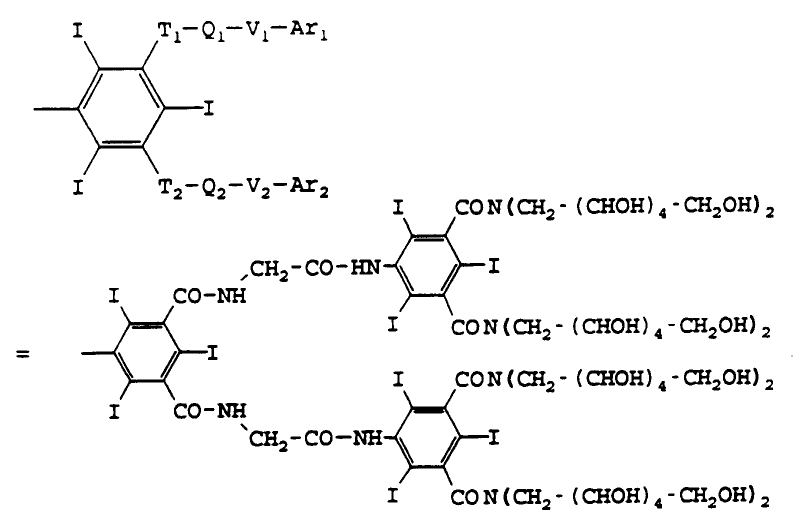
(1) Acide tétraiodotéréphtalique
   On dissout 10 g d'acide téréphtalique dans 80 g d'oléum contenant 60% d'anhydride sulfurique à 100° C. Après 1 heure à cette température, on ajoute 40 g d'iode par petites portions puis on élève la température jusqu'à 180° C et on la maintient pendant environ 5 heures avant de verser le milieu, refroidi, dans 3 volumes d'eau glacée. Le précipité formé est dissous dans une solution aqueuse de NaOH et le solide résiduel, essentiellement constitué d'hexa-iodobenzène, est isolé. Le diacide est reprécipité par acidification du filtrat et purifié par traitement au méthanol bouillant.
   Cristaux beiges. Rendement 30%.
(2) Dichlorure de l'acide tétraiodotéréphtalique
   10 g du diacide, 1,3 ml de diméthylformamide et 170 ml de SOCl₂ sont maintenus 15 heures à la température de reflux. Après concentration sous pression réduite, on introduit de l'éther de pétrole sur le résidu pour le faire cristalliser.
   On isole ainsi 10 g de solide
   CCM: Rf = 0,88 (conditions de l'exemple 1-1).
(3) Composé No. 3
   16 g du composé C₂ obtenu dans la préparation B, 4 g du dichlorure de l'acide tétraiodotéréphtalique et 1,5 ml de triéthylamine sont introduits dans 120 ml de diméthylformamide et le milieu réactionnel est maintenu 12 heures à 80° C. le précipité est éliminé par filtration après refroidissement et le solvant est évaporé sous pression réduite. Le résidu, dissous dans 1 litre d'eau, est soumis à une dia-ultrafiltration avec une cassette Minisette®, avec une membrane de seuil de coupure 5 kdaltons pour donner le produit cherché.
   CES: temps de rétention du composé No. 3: 37,3 minutes et du composé de départ C₂: 38,7 minutes

### Exemple 4

Composé No. 4 de formule I, dans laquelle 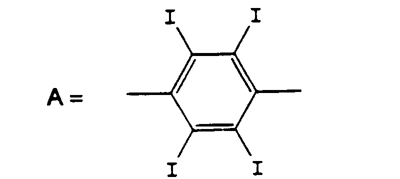 T₀-Q₀-V₀ = T'₀-Q'₀-V'₀ = CONH-CH₂-CONH et

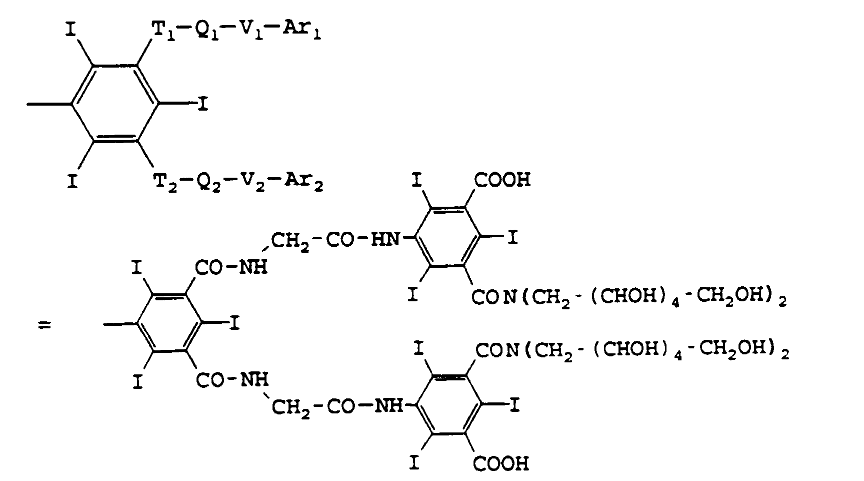

Etant donné la faible réactivité de l'acide carboxylique situé sur les noyaux phényles entre deux atomes d'iode, ce composé peut être préparé en appliquant le même mode opératoire que dans l'exemple 1.

### Exemple 5

Composé No. 5 de formule I, dans laquelle 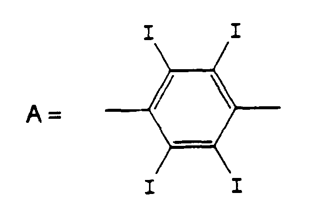 T₀-Q₀-V₀ = T'₀-Q'₀-V'₀ = CONH-CH₂-CONH et
T₁-Q₁-V₁-Ar₁ = T₂-Q₂-V₂-Ar₂ = CONH-CH₂-CONH-Ar

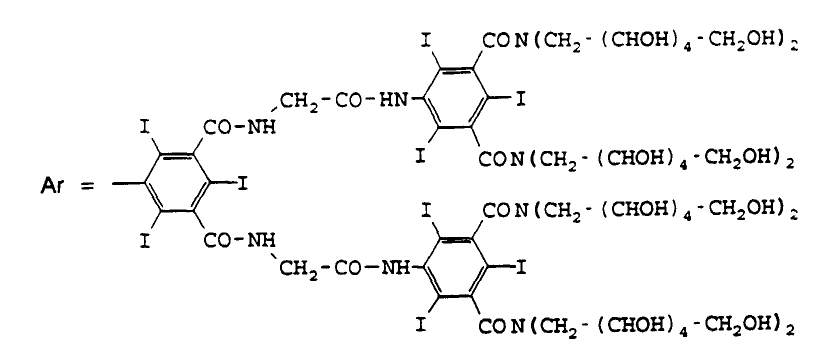

On applique le mode opératoire de l'exemple 2 pour préparer le composé No. 5 à partir du composé C₃. Sa mise en solution préalable dans le diméthylacétamide est avantageusement effectuée à 60° C.

On isole avec 50% de rendement le produit final.

### Exemple 6

Composé No. 6 de formule I, dans laquelle
A = -CH₂- ; T₀-Q₀-V₀ = T'₀-Q'₀-V'₀ = CONH-CH₂-CONH et
T₁-Q₁-V₁-Ar₁ = T₂-Q₂-V₂-Ar₂ = CONH-CH₂-CONH-Ar

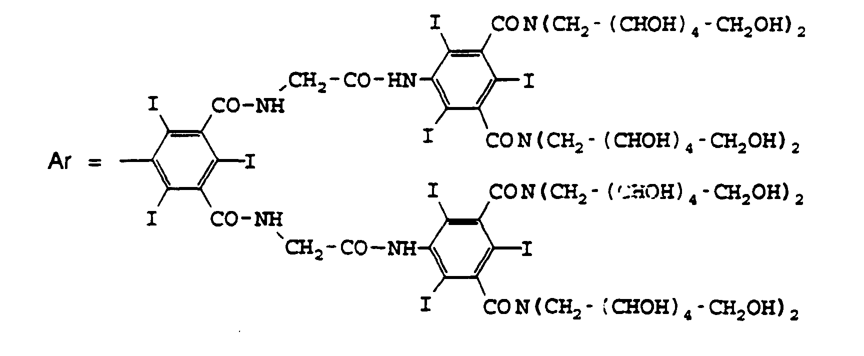

On introduit très lentement sous agitation vers 60° C 5,5 g de l'amine C₃ dans 20 ml de diméthylacétamide jusqu'à dissolution complète, puis 0,03 g de diacide, 0,11 g de 1-hydroxybenzotriazole, 0,16 g de chlorhydrate de 1-(3-diméthylaminopropyl)-3-éthylcarbodimide et 0,13 ml de triéthylamine. Après 24 heures d'agitation, on ajoute 5 volumes d'eau et on effectue une dia-ultrafiltration avec une membrane de seuil de coupure de 3 kdaltons. Le rétentat est concentré sous pression réduite pour donner 1 g de poudre blanche.

CES: temps de rétention du composé No. 6: 34,4 minutes.

### Exemple 7

Composé No. 7 de formule I, dans laquelle
A = -(CH₂)₄- ; T₀-Q₀-V₀ = T'₀-Q'₀-V'₀ = CONH-CH₂-CONH et
T₁-Q₁-V₁-Ar₁ = T₂-Q₂-V₂-Ar₂ = CONH-CH₂-CONH-Ar

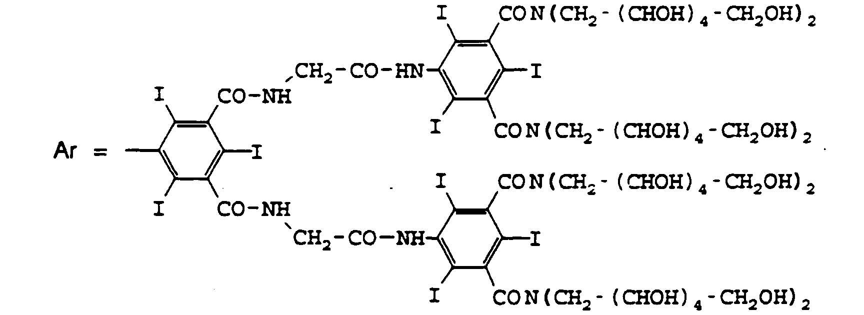

Le composé est préparé avec 55% de rendement en appliquant le mode opératoire décrit à l'exemple 6, en utilisant pour l'ultrafiltration une membrane de 3 kdaltons de seuil de coupure.

CES: temps de rétention: 34,4 minutes.

### Exemple 8

Composé No. 8 de formule I, dans laquelle 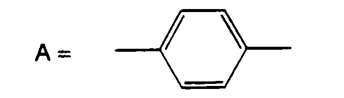 T₀-Q₀-V₀ = T'₀-Q'₀-V'₀ = CONH-CH₂-CONH et
T₁-Q₁-V₁-Ar₁ = T₂-Q₂-V₂-Ar₂ = CONH-CH₂-CONH-Ar

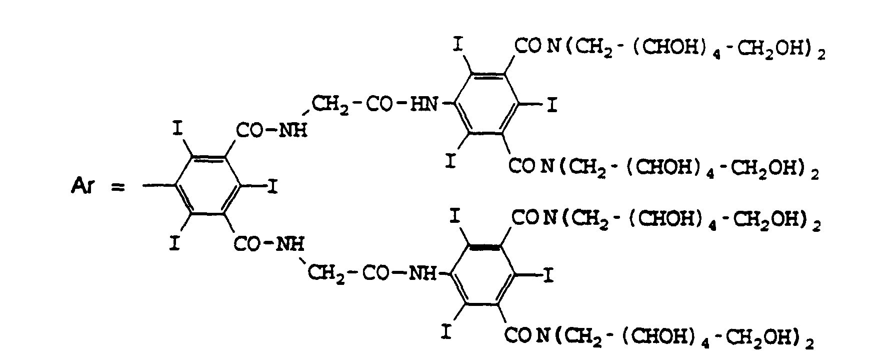

Le composé est préparé avec 50% de rendement en appliquant le mode opératoire décrit à l'exemple 6, en utilisant pour l'ultrafiltration une membrane de 3 kdaltons de seuil de coupure.

CES: temps de rétention: 34,3 minutes.

### Exemple 9

Composé No. 9 de formule I, dans laquelle
A = -CH₂- ; T₀-Q₀-V₀ = T'₀-Q'₀-V'₀ = CONH-CH₂-CONH et

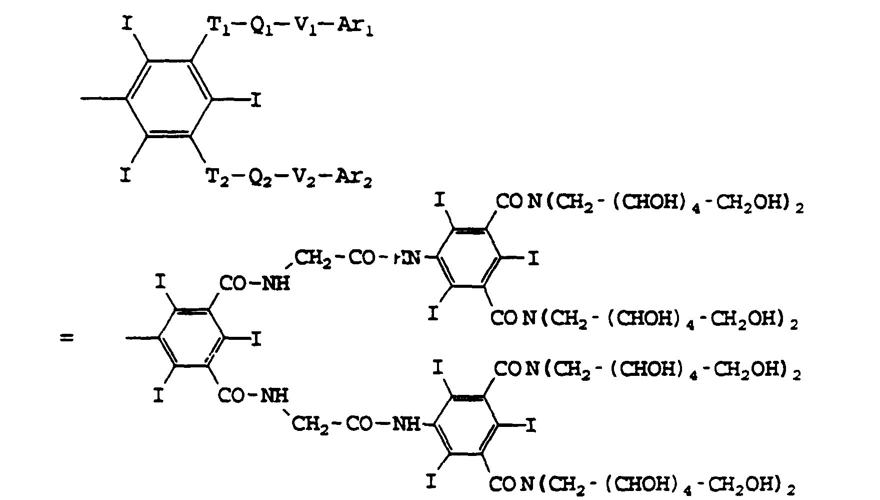

Ce composé est préparé, avec 48% de rendement, en appliquant le mode opératoire décrit à l'exemple 6, mais avec l'amine C₂ dissoute vers 20° C et après ultrafiltration avec une membrane de 1 kdalton de seuil de coupure.

CES: temps de rétention du composé No. 9: 36,3 minutes et du composé de départ C₂: 38,7 minutes.

### Exemple 10

Composé No. 10 de formule I, dans laquelle
A = -(CH₂)₄- ; T₀-Q₀-V₀ = T'₀-Q'₀-V'₀ = CONH-CH₂-CONH et

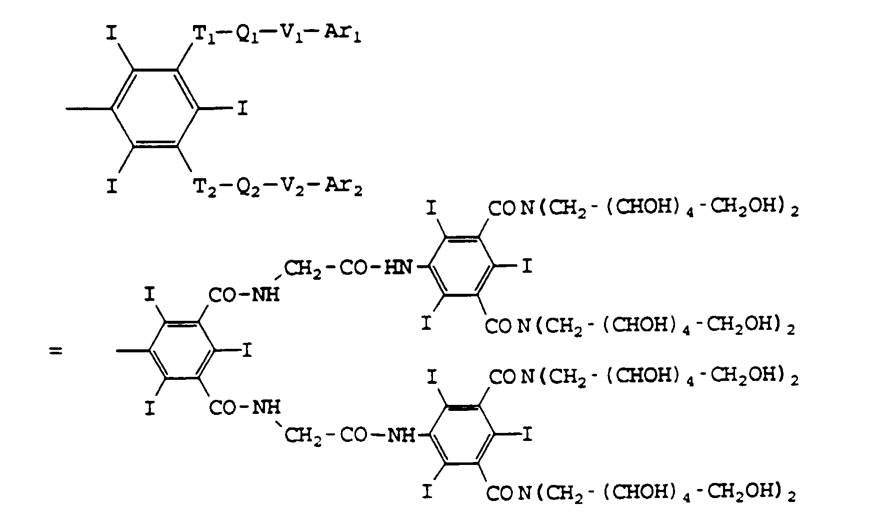

Le composé est préparé avec 52% de rendement en appliquant le mode opératoire décrit à l'exemple 9, en utilisant pour l'ultrafiltration une membrane de 1 kdalton de seuil de coupure.

CES: temps de rétention: 36,2 minutes.

### Exemple 11

Composé No. 11 de formule I, dans laquelle 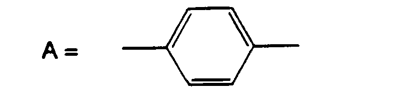 T₀-Q₀-V₀ = T'₀-Q'₀-V'₀ = CONH-CH₂-CONH et

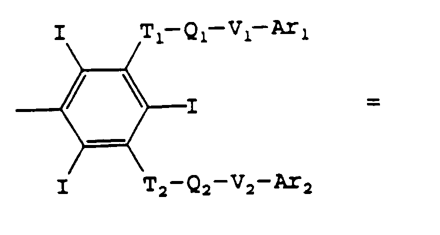

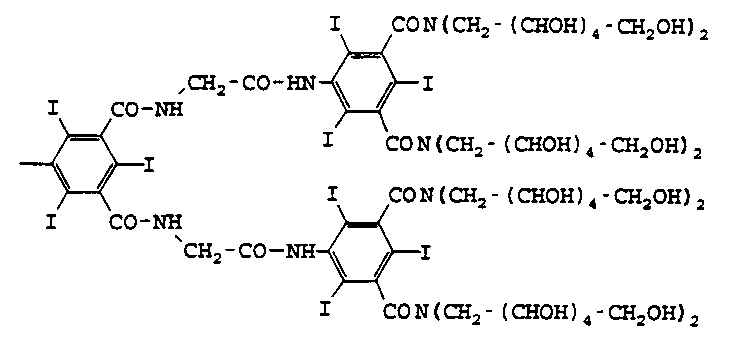

Le composé est préparé avec 48% de rendement en appliquant le mode opératoire décrit à l'exemple 9, en utilisant pour l'ultrafiltration une membrane de 1 kdalton de seuil de coupure.

CES: temps de rétention: 36,3 minutes.

## Revendications

1. Composés de formule 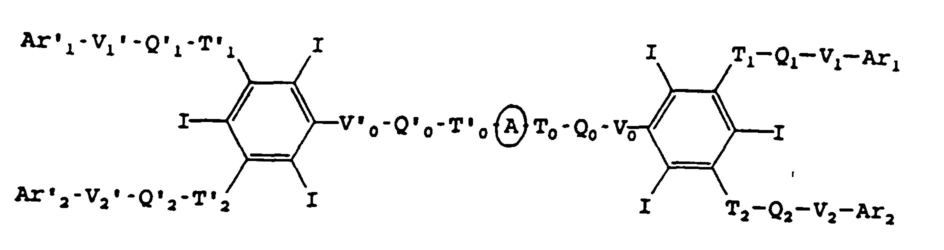 dans laquelle
- Tᵢ, Vᵢ, T'ᵢ, V'ᵢ, identiques ou différents, avec i = 0, 1, 2, sont choisis parmi les groupes O, CO-ND ou ND-CO, D pouvant être H, alkyle en C₁ à C₆, hydroxyalkyle ou polyhydroxyalkyle en C₁ à C₆,
- Qᵢ et Q'ᵢ, identiques ou différents, avec i = 0, 1, 2, représentent alkylène en C₁ à C₆, hydroxyalkylène ou polyhydroxyalkylène en C₁ à C₆,
- Arᵢ et Ar'ᵢ, identiques ou différents, avec i = 1, 2, représentent
· soit la formule II 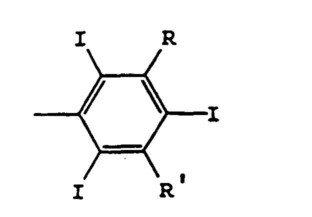 dans laquelle
R est COOH et R' est CONR'₁R'₂ ou N(R'₁)COR'₂,
R'₁ et R'₂ étant choisis parmi H, alkyle en C₁ à C₄, hydroxyalkyle ou polyhydroxyalkyle en C₁ à C₈, de telle sorte que NR'₁R'₂ comporte plus de 4 hydroxyles, ou R et R', identiques ou différents, représentent CONR'₁R'₂ ou N(R'₁)COR'_{2.},R'₁ et R'₂ étant choisis parmi H, alkyle en C₁ à C₄, hydroxyalkyle ou polyhydroxyalkyle en C₁ à C₈, de telle sorte que R'₁ et R'₂ comportent à eux deux plus de 8 hydroxyles ;
soit la formule III 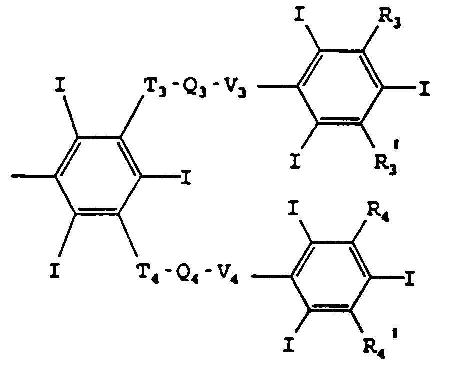 dans laquelle
les Tᵢ, Qᵢ Vᵢ, avec i = 3, 4, peuvent avoir rune des significations du cas où i = 0 et Rᵢ et R'ᵢ avec i 3, 4, peuvent avoir l'une des significations de R et R' de la formule II ou représentent T-Q-V-Ar, Ar étant représenté parla formule II et T, Q, V pouvant avoir les significations de Tᵢ, Qᵢ, Vᵢ,
et A est le reste biocompatible d'une molécule aliphatique ou aromatique de masse moléculaire inférieure à 1200, ayant deux liaisons libres susceptibles de donner avec T₀ et T'₀ un groupe amide ou étheroxyde, ainsi que les sels des bases pharmaceutiquement acceptables des acides.

2. Composés selon la revendication 1, caractérisés en ce que R et R' sont identiques et représentent CONR'₁ R'₂.

3. Composés selon la revendication 1, caractérisés en ce que lorsque R représente COOH, R'₁ et R'₂ comportent à eux deux plus de 6 hydroxyles.

4. Composés selon l'une des revendications précédentes caractérisés en ce que les deux groupes substituant A sont identiques.

5. Composés selon l'une des revendications précédentes caractérisés en ce que Tᵢ, Vᵢ, T'ᵢ, V'ᵢ sont des groupes CONH ou NHCO.

6. Composés selon l'une des revendications précédentes caractérisés en ce que Tᵢ-Qᵢ-Vᵢ, et T'ᵢ-Q'ᵢ-V'ᵢ avec i = 0 à 4, sont identiques et représentent CONH(CH₂)n-CONH avec n = 1 ou 2.

7. Composés selon l'une des revendications précédentes caractérisés en ce que Arᵢ et Ar'ᵢ avec i = 1, 2, sont identiques et représentés par la formule III dans laquelle Rᵢ et R'ᵢ avec i = 3, 4 sont identiques et ont les significations de R et R' de la formule II.

8. Composés selon rune des revendications précédentes caractérisés en ce que A est un noyau phényle, éventuellement substitué.

9. Composés selon l'une des revendications précédentes caractérisés en ce que T'₀-A-T₀ est un groupe -tétraiodotéréphtaloylamino ou téréphtaloylamino.

10. Composés selon rune des revendications 1 à 8 caractérisés en ce que T'₀-A-T₀ est un groupe-tétraiodoisophtaloylamino.

11. Composés selon l'une des revendications précédentes caractérisés en ce que T'₀-A-T₀ est un groupe HNCO(CH₂)nCONH dans lequel n = 1 à 4.

12. Compositions de produit de contraste pour imagerie médicale par rayons X, caractérisées en ce qu'elles comportent comme produit actif un composé selon l'une des revendications 1 à 11 associé un véhicule pharmaceutiquement acceptable.

13. Procédé de préparation des composés de formule I, qui consiste à faire réagir sur le reste A portant deux groupes choisis parmi COOH, COCI, OH, halogène, sulfonate ou NH₂:
- soit P^{α}₀-Q₀-V^{α}₀,
- soit 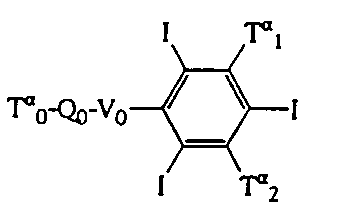 où α indique que le groupe est un groupe précurseur des Tᵢ et Vᵢ, qui est selon les cas une fonction amine, alcool ou phénol, acide ou chlorure d'acide, pour former un groupe amide ou étheroxyde,
et à répéter les réactions d'amidification et/ou d'éthérification sur le composé obtenu pour obtenir de proche en proche le composé de formule I,
- soit le composé de formule 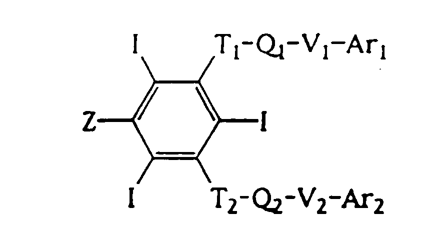 dans laquelle Z est un groupe précurseur de T₀-Q₀-V₀,
ou lorsque Z est un groupe précurseur de V₀ après avoir fixé sur A, T₀-Q₀-V^{α}₀, V^{α}₀ étant un groupe précurseur de V₀.

## Patentansprüche

1. Verbindungen der Formel 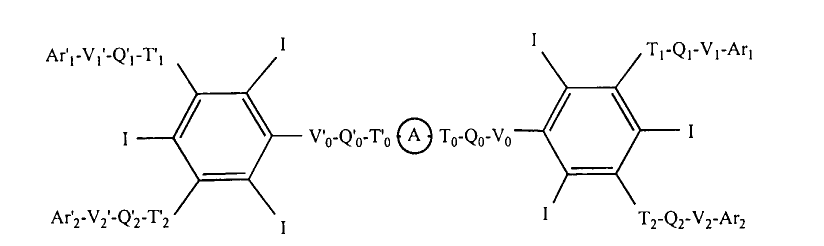
worin Tᵢ, Vᵢ, T'ᵢ, V'ᵢ identisch oder verschieden mit i = 0, 1, 2 sind und ausgewählt sein können aus den Gruppen O, CO-ND oder ND-CO, D, H, C₁ bis C₆-Alkyl, Hydroxyalkyl oder ein Polyhydroxyalkyl C₁ bis C₆-Alkyl,
Qᵢ und Q'ᵢ identisch oder verschieden sind mit i = 0, 1, 2 und ein C₁ bis C₆-Alkylen, Hydroxyalkylen oder Polyhydrox-C₁ bis C₆-Alken bedeuten,
Arᵢ und Ar'ᵢ identisch oder verschieden sind mit i = 1, 2 und entweder bedeuten
· die Formel II 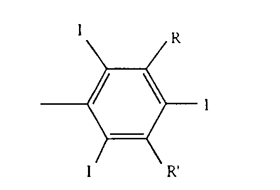 worin R COOH ist und R' CONR'₁R'₂ oder N(R'₁)COR'₂ ist, R'₁ und R'₂ ausgewählt sind aus H, C₁ bis C₄-Alkyl, C₁ bis C₈-Hydroxy- oder Polyhydroxyalkyl der Art, daß NR'₁R'₂ mehr als 4 Hydroxylgruppen aufweist, oder R und R' identisch oder verschieden sind und CONR'₁R'₂ oder N(R'1)COR₂ bedeuten, R'₁ und R'₂ ausgewählt sind aus H, C₁ bis C₄-Alkyl, C₁ bis C₈-Hydroxy- oder Polyhydroxyalkyl der Art, daß R'₁ und R'₂ mehr als 8 Hydroxylgruppen tragen;
· oder die Formel III 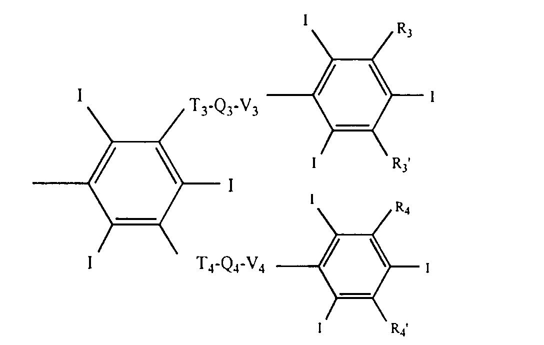 worin Tᵢ, Qᵢ, Vᵢ mit i = 3, 4 eine der Bedeutungen des Falles haben kann, in dem i = 0 ist, und Rᵢ und R'ᵢ mit i = 3, 4 eine der Bedeutungen von R und R' der Formel II haben können oder T-Q-V-Ar bedeuten, worin Ar durch die Formel II wiedergegeben wird und T, Q, V die Bedeutungen von T_{i,} Qᵢ, Vᵢ haben können
und A ein biologisch verträgliches aliphatisches oder aromatisches Molekül mit einem Molekulargewicht von kleiner als 1200 ist und zwei freie Bindungen aufweist, die mit T₀ und T'₀ eine Amidgruppe oder Etheroxyd ergeben können sowie Salze auf der Basis pharmazeutisch verträglicher Säuren.

2. Verbindungen nach Anspruch 1, **dadurch gekennzeichnet,** daß R und R' identisch sind und CONR'₁R'₂ bedeuten.

3. Verbindungen nach Anspruch 1, **dadurch gekennzeichnet**, daß in dem Fall, in dem R COOH bedeutet, R'₁ und R'₂ gemeinsam mehr als 6 Hydroxylgruppen aufweisen.

4. Verbindungen nach einem der vorhergehenden Ansprüche, **dadurch gekennzeichnet**, daß die beiden A substituierenden Gruppen identisch sind.

5. Verbindungen nach einem der vorhergehenden Ansprüche, **dadurch gekennzeichnet**, daß T_{i,} Vᵢ, T'ᵢ, V'ᵢ die Gruppen CONH oder NHCO sind.

6. Verbindungen nach einem der vorhergehenden Ansprüche, **dadurch gekennzeichnet**, daß Tᵢ-Qᵢ-Vᵢ und T'ᵢ-Q'ᵢ-V'ᵢ mit 0 bis 4 identisch sind und CONH(CH₂)n-CONH mit n = 1 oder 2 bedeuten.

7. Verbindungen nach einem der vorhergehenden Ansprüche, **dadurch gekennzeichnet**, daß Arᵢ und Ar'ᵢ mit i = 1, 2 identisch sind und durch die Formel III wiedergegeben werden, in der Rᵢ und R'ᵢ mit i = 3, 4 identisch sind und die Bedeutungen von R und R' der Formel II haben.

8. Verbindungen nach einem der vorhergehenden Ansprüche, **dadurch gekennzeichnet,** daß A ein gegebenenfalls substituierter Phenylkern ist.

9. Verbindungen nach einem der vorhergehenden Ansprüche, **dadurch gekennzeichnet**, daß T'₀-A-T₀ eine Tetraiodterephthaloylamino- oder Terephthaloylaminogruppe ist.

10. Verbindungen nach einem der Ansprüche 1 bis 8, **dadurch gekennzeichnet**, daß T'₀-A-T₀ eine Tetraiodisophthaloylaminogruppe ist.

11. Verbindungen nach einem der vorangegangenen Ansprüche, **dadurch gekennzeichnet**, daß T'₀-A-T₀ eine HNCO(CH₂)ₙ-CONH-Gruppe ist, in der n = 1 bis 4 bedeutet.

12. Kontrastmittel zur Erstellung von medizinischen Bildern durch Röntgenstrahlen, **dadurch gekennzeichnet,** daß sie als wirksames Mittel eine Verbindung nach einem der Ansprüche 1 bis 11 gemeinsam mit einem pharmazeutisch verträglichen Träger enthalten.

13. Verfahren zur Herstellung der Verbindungen der Formel I, welches darin besteht, daß man mit dem Rest A der zwei Gruppen, ausgewählt aus COOH, COCl, OH, Halogen, Sulfonat oder NH₂, enthält entweder T^{α}₀-Q₀-V^{α}₀ oder 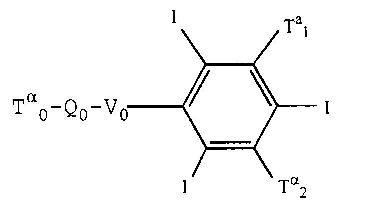 zur Bildung einer Amidgruppe oder Etheroxidgruppe reagieren läßt, worin α anzeigt, daß die Gruppe eine Vorstufengruppe von Tᵢ und Vᵢ ist, die je nach Fall eine Aminfunktion, ein Alkohol oder Phenol, Säure oder Chlorwasserstoffsäure ist und dem Wiederholen der Reaktionen der Amidbildung und/oder Etherbildung mit der erhaltenen Verbindung, um Stufe für Stufe die Verbindung der Formel I zu erhalten,
oder mit einer Verbindung der Formel 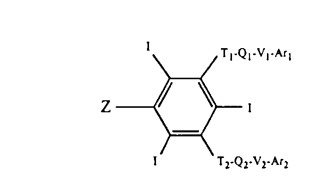 in der Z eine Vorstufengruppe von T₀-Q₀-V₀ ist,
oder wenn Z eine Vorstufengruppe von V₀ nach Bindung an A ist, T₀-Q₀-V^{α}₀, V^{α}₀ Vorstufengruppen von V₀ sind.

## Claims

1. Compounds having formula 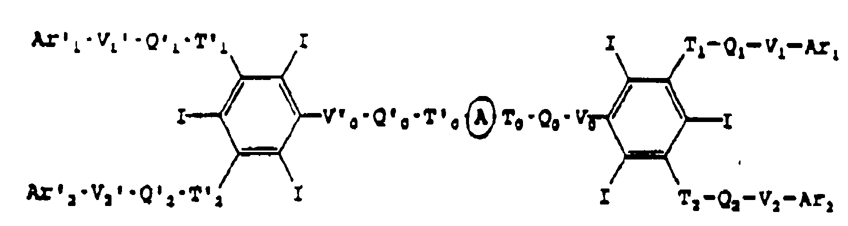 wherein
- Tᵢ, Vᵢ, T'ᵢ, V'ᵢ, being the same or different, where i=0, 1, 2, are selected from among the O, CO-ND or ND-CO groups, where D may be H, C₁₋₆ alkyl, C₁₋₆ hydroxyalkyl or polyhydroxyalkyl,
- Qᵢ and Q'ᵢ, being the same or different, where i=0, 1, 2, are C₁₋₆ alkylene or C₁₋₆ hydroxyalkylene or polyhydroxyalkylene,
- Arᵢ and Ar'ᵢ, being the same or different, where i=1, 2, are
- either formula II 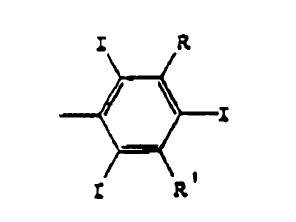 wherein
R is COOH and R' is CONR'₁R'₂ or N(R'₁)COR'₂,
R'₁ and R'₂ being selected from among H, C₁₋₄ alkyl, C₁₋₈ hydroxyalkyl or polyhydroxyalkyl such that NR'₁R'₂ has more than 4 OH, where R and R', being the same or different, are CONR'₁R'₂ or NR(R'₁)COR'₂, R'₁ and R'₂ being selected from among H, C₁₋₄ alkyl, C₁-C₈ hydroxyalkyl or polyhydroxyalkyl such that R'₁ and R'₂ together comprise more than 8 OH;
- or formula III 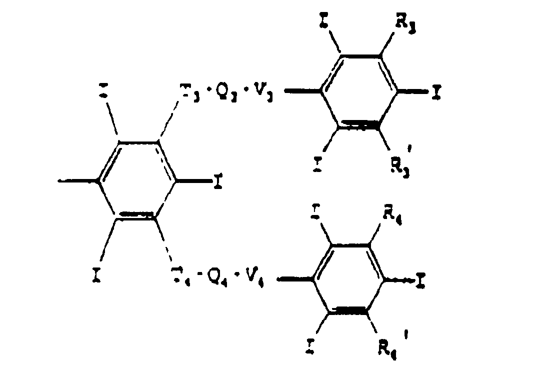 wherein
the Tᵢ, Qᵢ, Vᵢ, where i = 3, 4 may have one of the meanings whereby i=0 and Rᵢ and R'ᵢ, where i = 3, 4, may have one of the meanings of R and R' of formula II or are T-Q-V-Ar, Ar being formula II and it being possible for T, Q, V to have the meanings of Tᵢ, Qᵢ, Vᵢ,
and A is the biocompatible residue of an aliphatic or aromatic molecule having a molar mass of less than 1200 and having two free bonds capable of forming an amide or etheroxide group with T₀ and T'₀, as well as the salts of pharmaceutically acceptable bases of acids.

2. Compounds as claimed in claim 1, characterised in that R and R' are the same and are CONR'₁R'₂.

3. Compounds as claimed in claim 1, characterised in that if R is COOH, R'₁ and R'₂ together comprise more than 6 OH.

4. Compounds as claimed in one of the preceding claims, characterised in that the two substituent groups A are the same.

5. Compounds as claimed in one of the preceding claims, characterised in that Tᵢ, Vᵢ, T'ᵢ, V'ᵢ are CONH or NHCO groups.

6. Compounds as claimed in one of the preceding claims, characterised in that Tᵢ-Qᵢ-Vᵢ and T'ᵢ-Q'ᵢ-V'ᵢ where i= 0 to 4 are the same and represent CONH(CH₂)n-CONH where n= 1 or 2.

7. Compounds as claimed in one of the preceding claims, characterised in that Arᵢ and A'rᵢ where i = 1,2, are the same and are represented by formula III wherein Rᵢ and R'ᵢ where i = 3, 4 are the same and have the meanings of R and R' of formula II.

8. Compounds as claimed in one of the preceding claims, characterised in that A is a phenyl nucleus, optionally substituted.

9. Compounds as claimed in one of the preceding claims, characterised in that T'₀-A-T₀ is a tetraiodoterephthaloylamino or terephthaloylamino group.

10. Compounds as claimed in one of claims 1 to 8, characterised in that T'₀-A-T₀ is a tetraiodoisophthaloylamino group.

11. Compounds as claimed in one of the preceding claims, characterised in that T'₀-A-T₀ is a HNCO(CH₂)nCONH group wherein n = 1 to 4.

12. Contrast compounds for medical imaging by X-rays, characterised in that they contain a compound as claimed in one of claims 1 to 11 as the active product, in conjunction with a pharmaceutically acceptable vehicle.

13. A method of preparing compounds of formula I, which consists in causing a reaction on the residue A bearing two groups selected from among COOH, COCI, OH, halogen, sulphonate or NH₂:
- of either T^{α}₀-Q₀-V^{α}₀, or 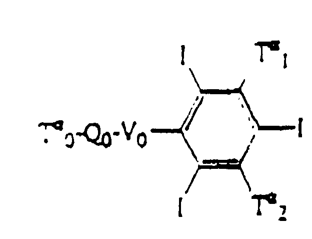 where α indicates that the group is a precursor of Tᵢ and Vᵢ which, as is the case, is an amine, alcohol or phenol, acid or chloric acid function, to form an amide or etheroxide group,
and repeating the amidification and/or etherification reactions on the compound obtained in order to obtain the compound of formula I gradually,
- or the compound of formula 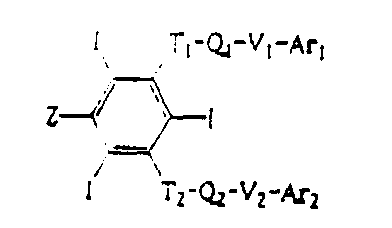 wherein Z is a precursor group of T₀-Q₀-V₀
or, if Z is a precursor group of V₀, after having fixed on A, of T₀-Q₀-V^{α}₀, V^{α}₀ being a precursor group of V₀.
